# EUROPEAN PATENT APPLICATION

(11) **EP 3 785 623 A1**
(43) Date of publication of application: **03.03.2021**
(21) Application number: 19194914.8
(22) Date of filing: 02.09.2019
(51) Int. Cl.: A61B 5/0482, A61B 5/00, A61B 5/16

(54) **SMART SYSTEM AND METHOD FOR MONITORING COGNITION STATUS**

(71) Applicant: National Chung-Shan Institute of Science and Technology, 325 Taoyuan City (TW)
(72) Inventor: Ko, Li-Wei, 300 Hsinchu City (TW); Wu, Pei-Lun, 408 Taichung City (TW); Tzou, Heng-An, 300 Hsinchu City (TW); Chang, Yang, 413 Taichung City (TW); Lu, Yi-Chen, 110 Taipei City (TW); Tang, Shyh-Jian, 325 Taoyuan City (TW); Yeh, Chia-Lung, 324 Taoyuan City (TW); Lin, Pin-Jun, 264 Yilan County (TW); Ruan, Jia-Siang, 333 Taoyuan City (TW)
(74) Representative: Straus, Alexander

(57) **Abstract**

A smart system (10) for monitoring a cognition status of a user includes an electroencephalography, abbreviated to EEG, acquisition module (102), configured to capture a plurality of brainwave signals; an analog filter circuit (104), coupled to the EEG acquisition module (102) and configured to amplify the plurality of brainwave signals; an analog to digital, abbreviated to ADC, circuit (106), coupled to the analog filter circuit (104) and configured to transform the plurality of brainwave signals into a plurality of digital signals; and a digital signaling processing, abbreviated to DSP, circuit (108), configured to retrieve a plurality of characteristics from the plurality of digital signals to determine the cognition status of the user.

## Description

### Field of the Invention

The present disclosure relates to a smart system and method for monitoring cognition statuses, and more particularly, to a smart system and method capable of monitoring brainwave signals of a user in real-time for monitoring the cognition statuses.

### Background of the Invention

With the development of biomedical technology, facilities for processing physiological signals are widely developed and utilized, e.g. a brain-computer interface (BCI) device utilized for recognizing electroencephalography (EEG) for analysis. The electroencephalography is an electrophysiological monitoring method to record electrical activities of a brain, which may be collected from a scalp of a user through sensing electrodes. The electroencephalography may be auxiliary for diagnosing brain diseases, e.g. epilepsy. In conventional applications of BCI, the brainwave signals are captured by a brainwave cap and transmitted to a module or device for further processing/analyzing through a wired method or a wireless method.

However, a recording device and a processing/analyzing device for the brainwave signals are necessary to monitor the brainwave signals of the user in real-time, which is hard to efficiently execute the monitoring process. In addition, when the recorded brainwave signals are transmitted via the wireless method, e.g. Bluetooth® or WiFi, for processing/analyzing, the operating space is limited due to distance limitation or signal obstacles, such that the transmitted brainwave signals are incomplete or interfered during the transmission. Therefore, an improvement to the conventional technique is necessary.

### Summary of the Invention

The present disclosure provides a smart system and method for monitoring a cognition status, which is capable of simultaneously monitoring the brainwave signals of a user, so as to improve the user experience.

This is achieved by a smart system for monitoring a cognition status of a user according to the independent claim 1 or by a method for monitoring a cognition status of a user according to the independent claim 8 here below. The dependent claims pertain to corresponding further developments and improvements.

As will be seen more clearly from the detailed description following below, the claimed smart system for monitoring a cognition status of a user, comprises an electroencephalography (EEG) acquisition module, configured to capture a plurality of brainwave signals; an analog filter circuit, coupled to the EEG acquisition module and configured to amplify the plurality of brainwave signals; an analog to digital (ADC) circuit, coupled to the analog filter circuit and configured to transform the plurality of brainwave signals into a plurality of digital signals; and a digital signaling processing (DSP) circuit, configured to retrieve a plurality of characteristics from the plurality of digital signals to determine the cognition status of the user.

In another aspect, the claimed method for monitoring a cognition status of a user, comprises capturing a plurality of brainwave signals; amplifying the plurality of brainwave signals; transforming the plurality of brainwave signals into a plurality of digital signals; and retrieving a plurality of characteristics from the plurality of digital signals to determine the cognition status of the user.

### Brief Description of the Drawings

FIG. 1 is a schematic diagram of a smart system according to an embodiment of the present disclosure.
FIG. 2 is a side view of the smart system according to an embodiment of the present disclosure.
FIG. 3 is a top view of the smart system according to an embodiment of the present disclosure.
FIG. 4 is a schematic diagram of a method according to an embodiment of the present disclosure.

### Detailed Description

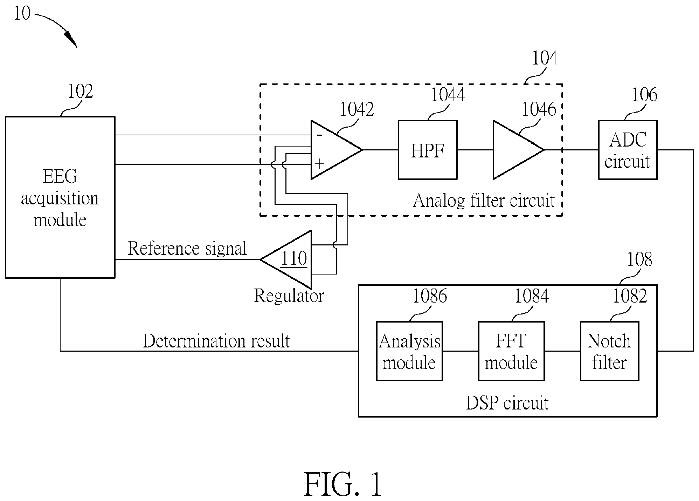

Please refer to FIG. 1, which is a schematic diagram of a smart system 10 according to an embodiment of the present disclosure. The smart system 10 may be a brainwave cap, and is utilized for monitoring a cognition status of a user. The smart system 10 includes an electroencephalography (EEG) acquisition module 102, an analog filter circuit 104, an analog to digital (ADC) circuit 106 and a digital signaling processing (DSP) circuit 108. The EEG acquisition module 102 is configured to capture a plurality of brainwave signals of the user. For example, the EEG acquisition module 102 may be a least a dry EEG electrode embedded in the brainwave cap to capture the brainwave signals of the user. The analog filter circuit 104 is coupled to the EEG acquisition module 102 and is configured to amplify the brainwave signals captured by the dry EEG electrode. The ADC circuit 106 is coupled to the analog filter circuit 104 and is configured to transform the brainwave signals into a plurality of digital signals. The DSP circuit 108 is configured to retrieve a plurality of characteristics from the digital signals to determine the cognition status of the user. Therefore, the smart system 10 of the present disclosure may simultaneously monitor the cognition or physiological status of the user by capturing the brainwave signals of the user.

In detail, the analog filter circuit 104 further includes a first amplifier 1042, a high pass filter 1044 and a second amplifier 1046. The first amplifier 1042 is configured to amplify the brainwave signals according to the brainwave signals from the EEG acquisition module 102 and a reference signal from a regulator 110, wherein the reference signal is a baseline of the brainwave signals. The high pass filter (HPF) 1044 is configured to filter out low frequency part of the brainwave signals. The second amplifier 1046 is configured to amplify the brainwave signals after the filtering procedure of the HPF 1044. Then, the brainwave signals are transformed into the digital signals by the ADC circuit 106.

The DSP circuit 108 further includes a notch filter 1082, a fast Fourier transform (FFT) module 1084 and an analysis module 1086. The notch filter 1082 is configured to perform filtering on the digital signals. The FFT module 1084 is configured to perform fast Fourier transform on the digital signals, and the analysis module 1086 is configured to retrieve the plurality of characteristics from the digital signals according to an algorithm, so as to determine the cognition status of the user. More specifically, the algorithm may be an artificial intelligence (AI) algorithm, e.g. a deep learning algorithm or a machine learning algorithm.

In an embodiment, the deep learning algorithm, e.g. a convolutional neural network (CNN) or a recurrent neural network (RNN), utilized for retrieving the characteristics of the digital signals is to collect the characteristics from the digital signals and to determine the cognition status of the user. In detail, the analysis module 1086 may collect the characteristics of the brainwave signals corresponding to different cognitive or physiological statuses of the user, such that the analysis module 1086 may extract or retrieve the characteristics from the digital signals based on the deep learning algorithm. Therefore, the analysis module 1086 may determine a determination result of the cognition status of the user based on characteristics retrieved by the deep learning algorithm and to trigger an alarm when the analysis module 1086 determines that the cognition status of the user is abnormal. Notably, the algorithm utilized by the analysis module 1084 to retrieve the plurality of characteristics from the digital signals is not limited to the deep learning algorithm; other algorithms which may be configured to retrieve the characteristics from the digital signals are also within the scope of the present disclosure.

In an embodiment, when one of the characteristic retrieved from the digital signals shows that the user is under extremely high pressure, too tired or too nervous, the analysis module 1086 may trigger the alarm or a notice on the brainwave cap to remind the user to take a rest, since the abnormal status of the user may likely lead to severe diseases.

Therefore, the smart system 10 is free from extra computers or processing devices for analyzing the brainwave signals, which makes the smart system 10 more convenient and efficient to monitor and recognize the cognition status of the user. Moreover, after the analysis module 1086 of the smart system 10 retrieves and analyzes the characteristics of the brainwave signals, a cognition model of the user may be built, which is utilized to determine the determination result, i.e. a current status of the user. As such, a neuro-feedback mechanism may simultaneously trigger a warning or a suggestion based on the determination result (i.e. the current status of the user) to monitor the cognition status of the user, so as to maintain the user's concentration and improve the efficiency when working on or enforcing tasks.

Notably, at least one of the EEG acquisition module 102, the analog filter circuit 104, the ADC circuit 106, the DSP circuit 108 and the regulator 110 of the present disclosure may be implemented on a system on a chip (SoC), and the SoC may be implemented on the smart system 10, i.e. the brainwave cap, to simultaneously determine the determination result of the cognition status of the user by the captured brainwave signals, and to trigger the alarm to remind the user to take a rest if necessary. In an example, a plurality of neuro-feedback speakers may be implemented on the smart system 10; more specifically, the neuro-feedback speakers may be implemented on the brainwave cap, such that the neuro-feedback speakers may be triggered when the smart system determines that the cognition or physiological status of the user is abnormal or the user is under too much pressure.

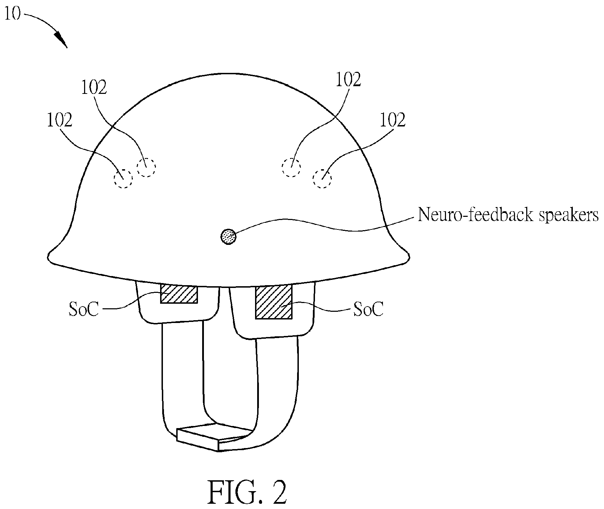

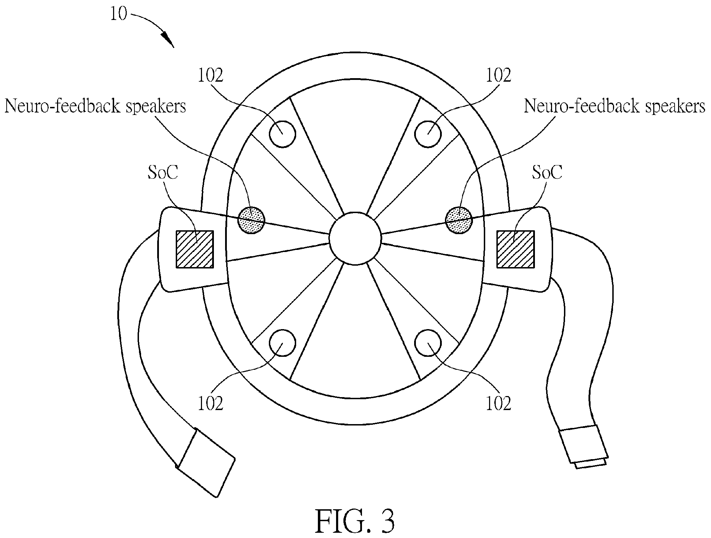

Please refer to FIG. 2 and FIG. 3. FIG. 2 is a side view of the smart system 10 according to an embodiment of the present disclosure. FIG. 3 is a top view of the smart system 10 according to an embodiment of the present disclosure. In FIGs. 2 and 3, the EEG acquisition module 102 may be multiple dry EEG electrodes, and the analog filter circuit 104, the ADC circuit 106, the DSP circuit 108 and regulator 110 may be implemented on a SoC. In addition, the neuro-feedback speakers may be implemented on lateral sides of the brainwave cap to warn the user when the cognition or physiological status of the user is abnormal. Notably, positions of the dry EEG electrodes, the SoC and the neuro-feedback speakers are not limited thereto, and may be adjusted or modified according to requirements of the user or the system.

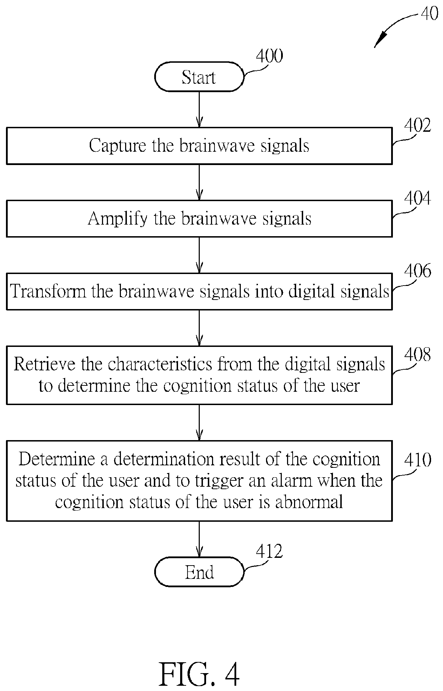

As to the method for monitoring the cognition status of the user, please refer to FIG. 4, which is a schematic diagram of a method 40 according to an embodiment of the present disclosure. The method 40 includes the following steps:
Step 400: Start.
Step 402: Capture the brainwave signals.
Step 404: Amplify the brainwave signals.
Step 406: Transform the brainwave signals into digital signals.
Step 408: Retrieve the characteristics from the digital signals to determine the cognition status of the user.
Step 410: Determine a determination result of the cognition status of the user and to trigger an alarm when the cognition status of the user is abnormal.
Step 412: End.

The operations of the method 40 may be referred to the above mentioned embodiments of the smart system 10, and are not narrated again for brevity.

Notably, the embodiments mentioned in the above are utilized for illustrating the concept of the present disclosure. Those skilled in the art may make modifications and alterations accordingly, and not limited herein. For example, the analog filter circuit 104 and the DSP circuit 108 may be realized by other elements with the same or similar function. Alternatively, the EEG acquisition module 102, the analog filter circuit 104, the ADC circuit 106, the DSP circuit 108 and the regulator 110 may be integrated on one SoC, and not limited thereto. The examples mentioned above are all within the scope of the present disclosure.

In summary, the present disclosure provides a smart system and method for monitoring the cognition status of the user capable of simultaneously monitoring and analyzing the brainwave signals of the user to maintain the concentration of the user and improve the working efficiency.

## Claims

1. A smart system (10) for monitoring a cognition status of a user, **characterized by** comprising:
an electroencephalography, abbreviated to EEG, acquisition module (102), configured to capture a plurality of brainwave signals;
an analog filter circuit (104), coupled to the EEG acquisition module (102) and configured to amplify the plurality of brainwave signals;
an analog to digital, abbreviated to ADC, circuit (106), coupled to the analog filter circuit (104) and configured to transform the plurality of brainwave signals into a plurality of digital signals; and
a digital signaling processing, abbreviated to DSP, circuit (108), configured to retrieve a plurality of characteristics from the plurality of digital signals to determine the cognition status of the user.

2. The smart system (10) of claim 1, **characterized in that** the analog filter circuit (104) comprises:
a first amplifier (1042), configured to amplify the plurality of brainwave signals according to the plurality of brainwave signals and a reference signal from a regulator (110), wherein the reference signal is a baseline of the plurality of brainwave signals;
a high pass filter (1044), configured to filter the plurality of brainwave signals; and
a second amplifier (1046), configured to amplify the plurality of filtered brainwave signals.

3. The smart system (10) of claim 1, **characterized in that** the DSP circuit (108) comprises:
a notch filter (1082), configured to perform filtering on the plurality of digital signals;
a fast Fourier transform, abbreviated to FFT, module (1084), configured to perform fast Fourier transform on the plurality of digital signals; and
an analysis module (1086), configured to retrieve the plurality of characteristics from the plurality of digital signals to determine the cognition status of the user according to an algorithm.

4. The smart system (10) of claim 3, **characterized in that** the algorithm is an artificial intelligence, abbreviated to Al, algorithm and is to collect the plurality of characteristics of the plurality of digital signals and to determine the cognition status of the user.

5. The smart system (10) of claim 3, **characterized in that** the analysis module (1086) determines a determination result of the cognition status of the user to trigger an alarm when the analysis module (1086) determines that the cognition status of the user is abnormal.

6. The smart system (10) of claim 1, **characterized in that** the smart system (10) is a brainwave cap.

7. The smart system (10) of claim 1, **characterized in that** the EEG acquisition module (102) comprises at least a dry electroencephalography electrode.

8. A method (40) for monitoring a cognition status of a user, **characterized by** comprising:
capturing a plurality of brainwave signals (402);
amplifying the plurality of brainwave signals (404);
transforming the plurality of brainwave signals into a plurality of digital signals (406); and
retrieving a plurality of characteristics from the plurality of digital signals to determine the cognition status of the user (408).

9. The method of claim 8, **characterized in that** the step of amplifying the plurality of brainwave signals comprises:
amplifying the plurality of brainwave signals according to the plurality of brainwave signals and a reference signal from a regulator (110), wherein the reference signal is a baseline of the plurality of brainwave signals;
filtering the plurality of brainwave signals; and
amplifying the plurality of filtered brainwave signals.

10. The method of claim 8, **characterized in that** the step of retrieving the plurality of characteristics from the plurality of digital signals to determine the cognition status of the user comprising:
performing filtering on the plurality of digital signals;
performing fast Fourier transform on the plurality of digital signals; and
retrieving the plurality of characteristics from the plurality of digital signals to determine the cognition status of the user according to an algorithm.

11. The method of claim 10, **characterized in that** the algorithm is an artificial intelligence, abbreviated to AI, algorithm and is to collect the plurality of characteristics of the plurality of digital signals and to determine the cognition status of the user.

12. The method of claim 10, **characterized by** comprising:
determining a determination result of the cognition status of the user to trigger an alarm when the cognition status of the user is abnormal (410).

13. The method of claim 8, **characterized in that** the smart system (10) is a brainwave cap.

14. The method of claim 8, **characterized in that** the plurality of brainwave signals are captured by at least a dry electroencephalography electrode.
